(19) Europäisches Patentamt
European Patent Office
Office européen des brevets

(11) **EP 3 376 465 A1**

(12) **EUROPEAN PATENT APPLICATION**
published in accordance with Art. 153(4) EPC

(43) Date of publication:
**19.09.2018 Bulletin 2018/38**

(21) Application number: **15908307.0**

(22) Date of filing: **12.11.2015**

(51) Int Cl.:
**G06Q 50/22** (2018.01)

(86) International application number:
**PCT/JP2015/081829**

(87) International publication number:
**WO 2017/081787 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **MORI, Tatsuya**
**Kawasaki-shi**
**Kanagawa 211-8588 (JP)**

• **UCHIDA, Daisuke**
**Kawasaki-shi**
**Kanagawa 211-8588 (JP)**
• **MAEDA, Kazuho**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake LLP**
**Lincoln House, 5th Floor**
**300 High Holborn**
**London WC1V 7JH (GB)**

(54) **FOOD INGESTION INDEX ESTIMATION DEVICE, FOOD INGESTION INDEX ESTIMATION METHOD, AND FOOD INGESTION INDEX ESTIMATION PROGRAM**

(57) An information processing apparatus includes a memory, and a processor coupled to the memory and configured to obtain time series data indicating a time-dependent change of a biological signal value after a meal, determine, based on the obtained time series data, a first feature amount of the time-dependent change of the biological signal value after the meal, determine, based on the determined first feature amount, an index value related to the meal, and output the determined index value.

FIG. 2A

FIG. 2B

## Description

TECHNICAL FIELD

The embodiment discussed herein is related to an information processing apparatus, a method, and a program.

BACKGROUND ART

A technology for estimating an index regarding ingestion has been desired. For example, a technology by which the amount of a meal is estimated in such a manner that an increase of a heart rate during a meal is focused is disclosed. There is Japanese National Publication of International Patent Application No. 10-504739 as the related art.

SUMMARY

TECHNICAL PROBLEM

It is difficult to estimate an ingestion index with high accuracy by the technology described above.

SOLUTION TO PROBLEM

According to an aspect of the invention, an information processing apparatus includes obtaining portion that obtains a feature amount of time series data indicating a time-dependent change of a biological signal value after a meal regarding the time series data of the biological signal value based on the meal, and determining portion that determines, based on the feature amount obtained by the obtaining portion, an index value related to the meal.

ADVANTAGEOUS EFFECTS OF INVENTION

The estimated accuracy of an ingestion index may be enhanced.

BRIEF DESCRIPTION OF DRAWINGS

FIG. 1 is a block diagram illustrating the hardware configuration of an ingestion-index estimation apparatus according to a first embodiment.
FIG. 2A is a block diagram of functions implemented by running a meal detection program, FIG. 2B is a functional block diagram representing the functions of a feature-vector calculation unit.
FIG. 3A is a graph illustrating meal-induced time variation of a heart rate, FIG. 3B is a graph illustrating feature points.
FIGs. 4A and 4B are each a graph illustrating area feature amounts.

FIG. 5A is a graph illustrating speed feature amounts, FIG. 5B is a graph illustrating amplitude feature amounts, FIG. 5C is a graph illustrating time feature amounts.
FIGs. 6A and 6B are each a graph illustrating function feature amounts.
FIGs. 7A and 7B are graphs illustrating a method for calculating estimated calories of ingested food.
FIGs. 8A and 8B are graphs illustrating a method for calculating estimated remaining calories.
FIG. 9 is a graph illustrating a method for calculating estimated digestive power.
FIG. 10 is a diagram illustrating a flowchart representing calculation of a function f.
FIG. 11 is a diagram illustrating a flowchart representing estimation of a caloric intake.
FIG. 12 is a diagram illustrating a flowchart representing a process for calculating an area II.
FIG. 13 is a graph illustrating a different example of meal-induced time variation of a heart rate.
FIGs. 14A and 14B are each a diagram illustrating a different apparatus configuration of the ingestion-index estimation apparatus.

DESCRIPTION OF EMBODIMENT

(First Embodiment)

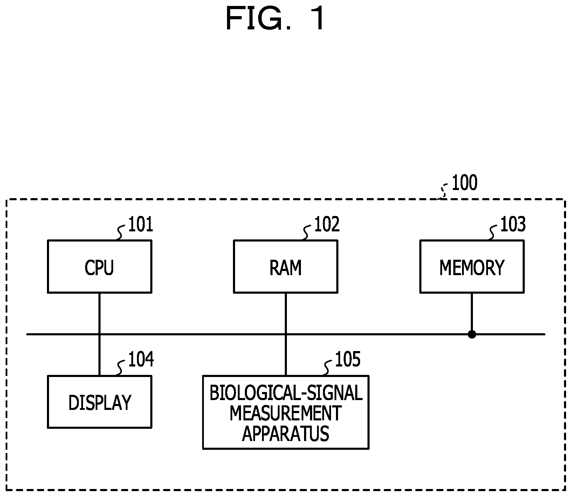

FIG. 1 is a block diagram illustrating the hardware configuration of an ingestion-index estimation apparatus 100 according to a first embodiment. As illustrated in FIG. 1, the ingestion-index estimation apparatus 100 includes a central processing unit (CPU) 101, a random access memory (RAM) 102, a memory 103, a display 104, a biological-signal measurement apparatus 105, and other devices. These devices are coupled to each other via a bus.

The CPU 101 is a central processing unit. The CPU 101 includes one or more cores. The RAM 102 is a volatile memory that temporarily stores a program run by the CPU 101, data processed by the CPU 101, and the like.

The memory 103 is a nonvolatile memory. For example, a read only memory (ROM), a solid state drive (SSD) such as a flash memory, a hard disk driven by a hard disk drive, or the like is usable as the memory 103. The memory 103 stores therein an ingestion-index estimation program according to this embodiment. The display 104 is a liquid crystal display, an electroluminescence panel, or the like and displays the result of an ingestion-index estimation process described later.

The biological-signal measurement apparatus 105 is an apparatus that measures biological signal values of living things that eat, such as humans and animals. Examples of the biological signal values include a blood pressure, a body temperature, an electric skin resistance, an electrocardiographic complex, a pulse wave form, a heart rate (pulse rate), and a skin temperature. In this embodiment, for example, the biological-signal measurement apparatus 105 is an apparatus that measures the heartbeat (pulse) of a user. For example, the biological-signal measurement apparatus 105 may be an electrocardiograph, a pulsation sensor, or another apparatus.

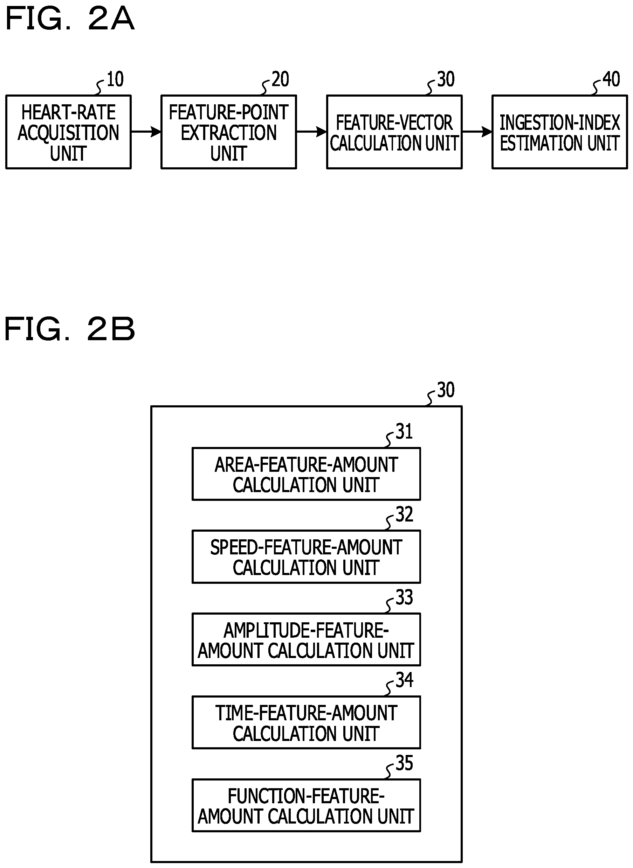

The ingestion-index estimation program stored in the memory 103 is loaded on the RAM 102 to be able to be run. The CPU 101 runs the ingestion-index estimation program loaded on the RAM 102. The ingestion-index estimation apparatus 100 thereby executes processes. FIG. 2A is a block diagram of functions implemented by the ingestion-index estimation program. By running the ingestion-index estimation program, a heart-rate acquisition unit 10, a feature-point extraction unit 20, a feature-vector calculation unit 30, an ingestion-index estimation unit 40, and other components are implemented.

FIG. 2B is a functional block diagram representing the functions of the feature-vector calculation unit 30. As illustrated in FIG. 2B, the feature-vector calculation unit 30 functions as an area-feature-amount calculation unit 31, a speed-feature-amount calculation unit 32, an amplitude-feature-amount calculation unit 33, a time-feature-amount calculation unit 34, a function-feature-amount calculation unit 35, and other components.

(Ingestion-index Estimation Process)

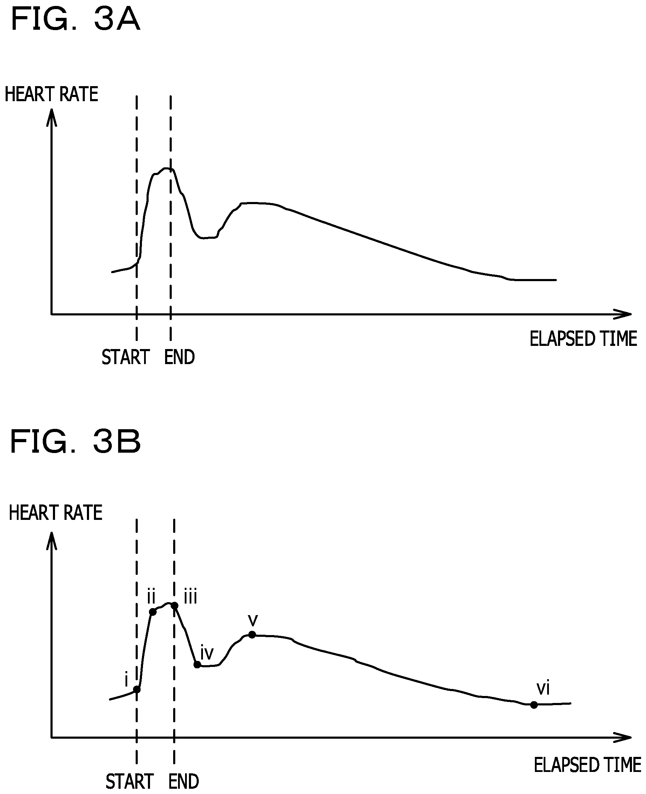

The heart-rate acquisition unit 10 acquires heartbeat from the biological-signal measurement apparatus 105 and thereby acquires time variation of a heart rate. FIG. 3A illustrates meal-induced time variation of a heart rate. In FIG. 3A, the horizontal axis represents elapsed time, and the vertical axis represents heart rate. The heart rate is a pulsation rate per unit time and is specifically a pulsation rate per minute. Hereinafter, the term "heart rate" denotes a pulsation rate per minute unless otherwise particularly stated.

As illustrated in FIG. 3A, two peaks appear in a meal-induced heart-rate-rising section. The first peak represents a heart rate change appearing immediately after the start of a meal. The second peak represents a heart rate change appearing over a long span of time from time of the meal. For example, the first peak is supposed to be attributed to mastication, swallowing, hand movement, and the like. The second peak is supposed to be attributed to movement of digestive organs such as a digestive event and absorption. The start time point of the rising section at which the rising edge of the first peak is detected is a meal-start time point. For example, a start time point at which the rising speed of the heart rate becomes greater than or equal to a threshold and a rising range becomes greater than or equal to a threshold may be detected as the rising edge. The local maximum point of the first peak is a meal-end time point. A time point at which the heart rate in the second peak returns to a predetermined value after exceeding the local maximum point is an end time point of the meal-induced heart-rate-rising section. For example, a time point at which the heart rate in the second peak returns to the heart rate observed at the start of the meal may be used as the end time point of the heart-rate-rising section, or a time point at which the heart rate returns to a value obtained by adding or subtracting a predetermined value to or from the heart rate observed at the meal-start time point may be used as the end time point of the heart-rate-rising section. The wave form of the heart rate from the start time point of the meal-induced heart-rate-rising section to the end time point is referred to as a heartbeat peak.

The feature-point extraction unit 20 extracts feature points for feature vector calculation in a heartbeat peak acquired by the heart-rate acquisition unit 10. First, as illustrated in FIG. 3B, the feature-point extraction unit 20 extracts a feature point i (normally corresponding to a meal start time) at time when the heart rate starts rising due to the influence of the meal. For example, the feature-point extraction unit 20 detects the aforementioned rising edge and thereby extracts the feature point i. Alternatively, a value manually input by the user may be used. Alternatively, since the heart rate is mentally influenced before an actual meal and thus starts rising in some cases, the minimum value of the heart rate within a predetermined time (for example, 15 minutes) before the actual meal time may be used as the feature point i.

The feature-point extraction unit 20 then extracts a feature point ii at time when the rising of the heart rate is settled (the rising speed is decreased) after the meal start. For example, the feature-point extraction unit 20 performs straight line fitting with the least squares method on data regarding a section from the feature point i to time t1 within a predetermined time (for example, within three minutes) after the feature point i. If an error between the fitted line and the actual data is less than or equal to a threshold, the feature-point extraction unit 20 updates the time t1 with t1+$\delta$t (for example, $\delta$t = 10 seconds). The feature-point extraction unit 20 repeats this process by using the updated time t1. The feature-point extraction unit 20 uses, as the feature point ii, time when the error exceeds the threshold.

The feature-point extraction unit 20 then extracts a feature point iii that is the local maximum point of the first peak of the heart rate after the meal start (time when the heart rate starts to lower). The feature point iii normally corresponds to a meal end time. However, a method using the local maximum point includes an error in some cases. Hence, data regarding a section from a point before the local maximum point to a point after the local maximum point (for example, data regarding a section from time five minutes before the local maximum point to time five minutes after the local maximum point) may be acquired, the time series data of the heart rate may be divided into two segments by using the bottom-up algorithm, and then time at the border of the segments may be extracted as the feature point iii. Alternatively, a manually input value may be used.

The feature-point extraction unit 20 then extracts a feature point iv at time when the lowering of the heart rate is settled (the lowering speed is decreased) in the first peak. For example, the feature-point extraction unit 20 may use a point a predetermined time after the feature point i (for example, after 30 minutes) as the feature point iv. Alternatively, the feature-point extraction unit 20 performs the straight line fitting with the least squares method on data regarding a section from time at the feature point iii to time t1 within a predetermined time (for example, within three minutes) after the feature point iii. If an error between the fitted line and the actual data is less than or equal to a threshold, the feature-point extraction unit 20 updates the time t1 with t1+δt (for example, δt = 10 seconds). The feature-point extraction unit 20 repeats this process by using the updated time t1. The feature-point extraction unit 20 uses, as the feature point iv, time when the error exceeds the threshold.

The feature-point extraction unit 20 then extracts, as a feature point v, time when a gentle decrease of a high level heart rate after the end of the meal is started. For example, the feature-point extraction unit 20 may extract the local maximum point of the second peak as the feature point v. Alternatively, the feature-point extraction unit 20 performs moving average in a 30-minute window on data regarding a section from time a predetermined time (for example, one hour) before the time acquired as the feature point i and time a predetermined time (for example, four hours) after the time acquired as the feature point i. Based on this, the feature-point extraction unit 20 may use, as the feature point v, time corresponding to the maximum value of the moving average data regarding a section within a predetermined time (for example, within one hour) after the time of the feature point iii.

The feature-point extraction unit 20 then extracts, as a feature point vi, time (the end time of the heartbeat peak) when the gentle decrease of the high level heart rate after the meal end ceases. For example, the feature-point extraction unit 20 performs the straight line fitting with the least squares method on data regarding a section from time at the feature point v to time t1 within a predetermined time (for example, within one hour) after the feature point v. If an error between the fitted line and the actual data is less than or equal to a threshold, the feature-point extraction unit 20 updates the time t1 with t1+δt (for example, δt = 10 seconds). The feature-point extraction unit 20 repeats this process by using the updated time t1. The feature-point extraction unit 20 uses, as the feature point vi, time when the error exceeds the threshold.

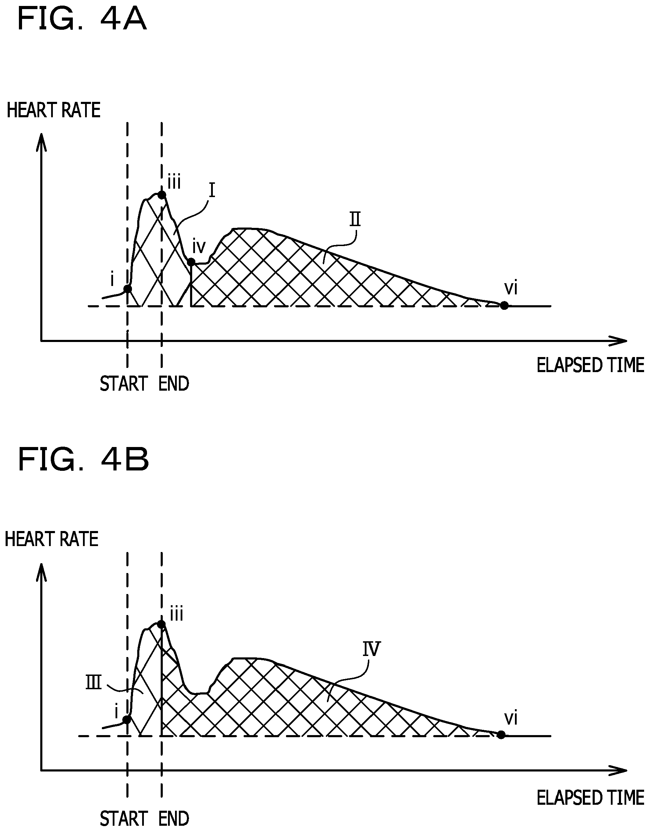

Next, the feature-vector calculation unit 30 calculates a feature vector related to ingestion by using the feature points extracted by the feature-point extraction unit 20. The feature vector includes one or more feature amounts. First, the area-feature-amount calculation unit 31 calculates area feature amounts. The area feature amounts include at least an integration value of the time variation of a biological signal value after the meal end. For example, as illustrated in FIG. 4A, the area-feature-amount calculation unit 31 calculates an area I of the first peak and an area II of the second peak by using integration. The area I is an area from the feature point i to the feature point iv. The area II is an area from the feature point iv to the feature point vi. Next, as illustrated in FIG. 4B, the area-feature-amount calculation unit 31 calculates an area III from the meal start to the meal end and a peak area IV from the meal end to a point where the heart rate change line becomes flat. The area III is an area from the feature point i to the feature point iii. The area IV is an area from the feature point iii to the feature point vi. The area-feature-amount calculation unit 31 then calculates an area V between any time and time in one of the areas I to IV. Note that the areas I to V are each the area of ΔHR that is an increase from a predetermined heart rate. The heart rate at the feature point i, the heart rate at the feature point vi, or the like may be used as the predetermined heart rate.

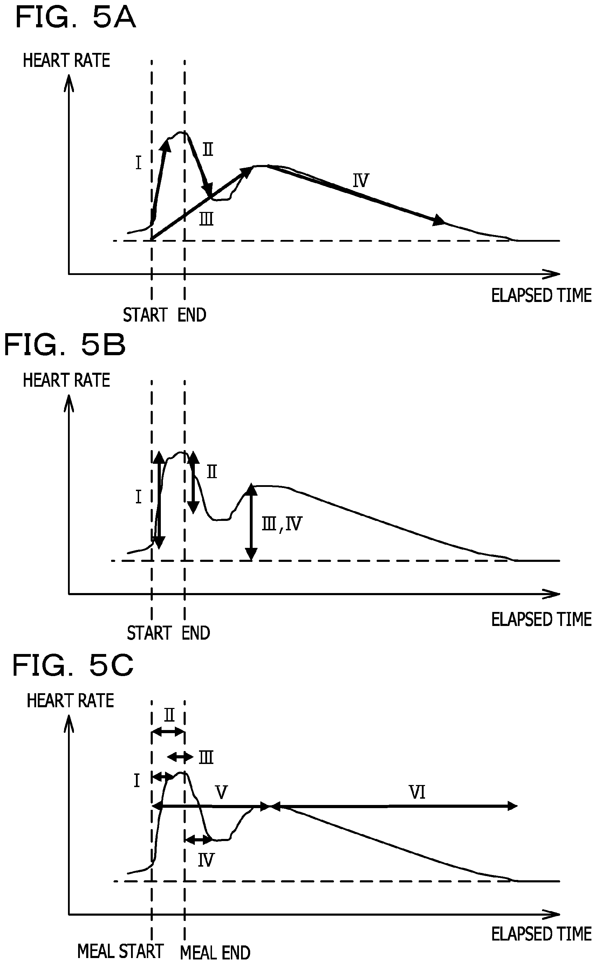

Next, as illustrated in FIG. 5A, the speed-feature-amount calculation unit 32 calculates speed feature amounts. The speed feature amounts include at least one of a rising speed and a lowering speed of at least a section between a point in time variation of the biological signal value and a point after the meal end in the time variation of the biological signal value. For example, the speed-feature-amount calculation unit 32 calculates a speed I at which the level of the heart rate becomes high and is kept unchanged to some extent after the meal start. The speed I is, for example, a rising speed of the heart rate from the feature point i to the feature point ii. The speed-feature-amount calculation unit 32 then calculates a speed II at which the level of the heart rate approaches, after the meal end, to the level of the heart rate observed before the meal start. The speed II is, for example, a lowering speed of the heart rate from the feature point iii to the feature point iv. The speed-feature-amount calculation unit 32 then calculates a speed III of rising from a point before the meal start to the second peak. The speed III is, for example, a rising speed calculated from a line connecting the feature point i and the feature point v. The speed-feature-amount calculation unit 32 then calculates a speed IV at which the raised level of the heartbeat observed after the meal end for a long time approaches to the original level observed before the meal start. The speed IV is, for example, a lowering speed of the heart rate in a section from the feature point v to the feature point vi. The speed-feature-amount calculation unit 32 then calculates a rising speed or a lowering speed of the heart rate at any time as a speed V.

Next, as illustrated in FIG. 5B, the amplitude-feature-amount calculation unit 33 calculates amplitude feature amounts. The amplitude feature amounts include at least one of a rising range and a lowering range of at least a section between a point in time variation of the biological signal value and a point after the meal end in the time variation of the biological signal value. For example, the amplitude-feature-amount calculation unit 33 calculates an amplitude I from the level of a heart rate observed at a point before the meal start to a high level observed at a point during the meal. The amplitude I corresponds to, for example, a rising range from the feature point i to the feature point ii. The amplitude-feature-amount calculation unit 33 then calculates an amplitude II between a heart rate at the meal end and a heart rate observed when the level of the heart rate approaches, after the meal end, to the level of the heart rate before the meal start. The amplitude II corresponds to, for example, a lowering range from the feature point iii to the feature point iv. The amplitude-feature-amount calculation unit 33 then calculates an amplitude III from a point before the meal start to the second peak. The amplitude III corresponds to, for example, a rising range from the feature point i to the feature point v. The amplitude-feature-amount calculation unit 33 then calculates an amplitude IV in which the level of a heart rate that is high and observed for a long time in the second peak approaches to the original level before the meal start and then becomes steady. The amplitude IV corresponds to, for example, a lowering range from the feature point v to the feature point vi. The amplitude-feature-amount calculation unit 33 then calculates an amplitude V from a point before the meal start to any time.

Next, as illustrated in FIG. 5C, the time-feature-amount calculation unit 34 calculates time feature amounts. The time feature amounts include at least a time length between a point in time variation of the biological signal value and a point after the meal end in the time variation of the biological signal value. For example, the time-feature-amount calculation unit 34 calculates a time period I from the meal start to a point when the heart rate is stabilized to the high level heart rate observed during the meal. The time period I corresponds to, for example, a time length from the feature point i to the feature point ii. The time-feature-amount calculation unit 34 then calculates a time period II from the meal start to the meal end. The time period II corresponds to, for example, a time length from the feature point i to the feature point iii. The time-feature-amount calculation unit 34 then calculates a time period III in which the level of the heart rate after the meal start becomes the high level observed during the meal. The time period III corresponds to, for example, a time period from the feature point ii to the feature point iii. The time-feature-amount calculation unit 34 then calculates a time period IV in which the level of the heart rate approaches, after the meal end, to the level before the meal start. The time period IV corresponds to, for example, a time period from the feature point iii to the feature point iv. The time-feature-amount calculation unit 34 then calculates a time period V from the meal start to the second peak. The time period V corresponds to, for example, a time length from the feature point i to the feature point v. The time-feature-amount calculation unit 34 then calculates a time period VI from the local maximum point of the second peak to the end of the second peak. The time period VI corresponds to, for example, a time length from the feature point v to the feature point vi. The time-feature-amount calculation unit 34 then calculates a time period VII between time used as one of the times I to VI and any time.

Next, the function-feature-amount calculation unit 35 calculates function feature amounts. Note that the meal-induced heart-rate variation over a long span of time is supposed to be caused by a plurality of factors. For example, digestion and absorption time varies with the nutrient, and it is thus conceivable that the heart rate variation varies as the result of this. Hence, in this embodiment, it is assumed that the heart-rate variation over a long span of time that is based on a heart rate and induced by a meal is dividable into functions on a per-factor basis, and the area feature amounts, the speed feature amounts, the amplitude feature amounts, the time feature amounts, or values similar to these are calculated as feature amounts. If occurrence of long-term heart rate variation caused by, for example, three factors is assumed, the conceivable way of dividing is as follows.

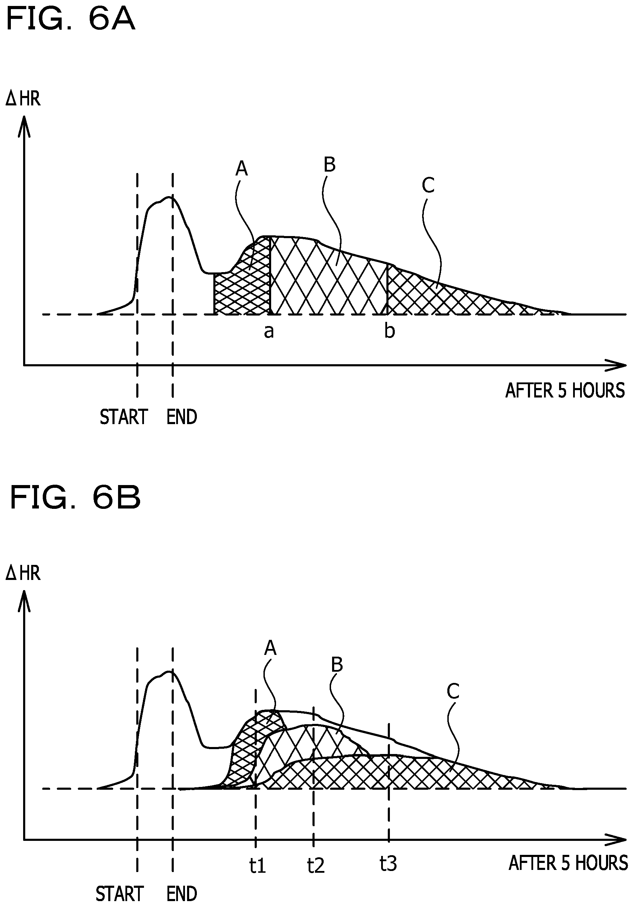

First, as illustrated in FIG. 6A, the second peak may be divided into three time ranges A to C. For example, the area of $\Delta HR(t)$ of a time range applying "the start time of the second peak (feature point iv) $\leq t < a$" may be used as the area of the time range A. The area of $\Delta HR(t)$ of a time range applying "$a \leq t < b$" may be used as the area of the time range B. The area of $\Delta HR(t)$ of a time range applying "$b \leq t <$ the end time of the second peak (feature point vi)" may be used as the area of the time range C. The area of each of the time ranges A to C may be used as an area feature amount.

Alternatively, as illustrated in FIG. 6B, the above-described three factors may be expressed as Gaussian functions, respectively, and parameters $p_1$ to $p_3$ expressed in accordance with the following formula may be determined to obtain the smallest error from data having the total of the three Gaussian functions. Note that the parameters $p_1$ to $p_3$ are $\Delta HR$ at times t1 to t3 at the peak points of the three Gaussian functions, respectively. The times t1 to t3 and $\sigma_1$ to $\sigma_3$ may have been determined based on previous knowledge. Note that the parameters pi to $p_3$ may be used as the amplitude feature amounts. If a motion section is acquirable based on an inertial sensor or the like, the time variation of the heart rate used for feature points i to vi or the feature vector calculation may be obtained by removing an influence of the motion from the heart rate in the motion section. Features of time variation of a heart rate that is induced by a meal to a greater extent may thereby be acquired. For example, it is conceivable that such processing as deleting heartbeat data in the motion section and then performing linear interpolation after the deletion is performed.

$$\Delta HR(t) = \sum_{i=1}^{3} p_i * \exp\left(-\frac{(t - t_i')^2}{2\sigma_i^2}\right)$$

The ingestion-index estimation unit 40 calculates an index (ingestion index) regarding the condition of a person, an eating behavior, ingested food, and the like and regarding a relationship thereamong by using the calculated feature vector. The ingestion index is, for example, calories related to food or metabolism. Further, examples of ingestion index include a caloric intake. The ingestion index may be expressed as a function of the feature vector. Accordingly, in a case where the feature vector is x, the ingestion index may be expressed as f(x). Note that a function f may be based on knowledge given in advance or may be a model built up based on a relationship with known data. The function f may also be prepared for a person, place, hour, or the like. For example, the function f may also be prepared separately for each of breakfast, lunch, dinner, and snacking. In addition, the ingestion index includes not only calories related to food or metabolism but also a bodily change related to a meal, a change of working of the brain or a bodily organ, a change of blood flow or a blood component, the content of a meal, the degree of content of an ingestion action, and the like. Specifically, as the ingestion index, digestibility, the degree of hunger, the amount of a meal, the degree of quick eating, the degree of slow eating, a nutrient intake, a blood sugar level, a body temperature rise, a calorific value, digestive organ movement, the degree of mastication, perspiration, a digestion load index, and the like are cited. Note that calories of ingested food include not only calories absorbed by the body but also calories caused by physical combustion of the ingested food and the like and may be estimated in such a manner that Atwater coefficients or the like is considered. When the ingestion index is calculated, the feature vector may be calculated by combining feature amounts derived from a plurality of pieces of biological information. Further, information other than a biological signal may be added to the feature vector.

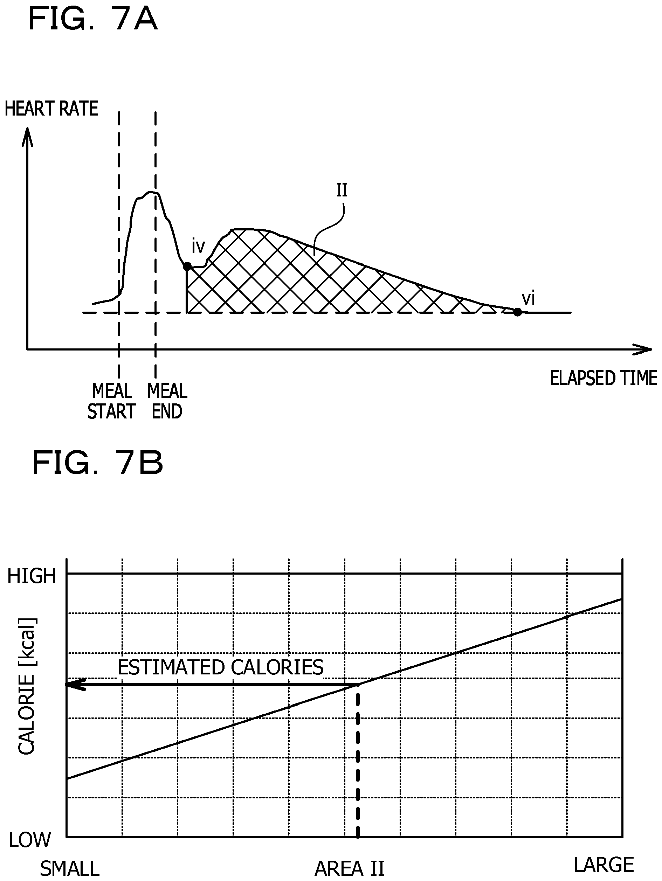

Subsequently, a specific example of a method for calculating estimated calories of ingested food will be described. For example, as illustrated in FIG. 7A, the area II of the second peak calculated by the area-feature-amount calculation unit 31 may be used as the feature vector x. For example, a relationship between the feature vector regarding a known meal and a caloric intake is learned from existing data by using a fitted curve. The learning fitted curve may be generated as the function f. FIG. 7B is a graph illustrating the function f. Estimated calories for unknown calories of a meal may be calculated from the feature vector x by using the generated function f.

A state varying with the progress of digestion may be reflected in the calories of ingested food. For example, $(x_1, x_2, x_3)$ = (the area of the time range A, the area of the time range B, and the area of the time range C) may be used as the feature vector x. The areas are illustrated in FIG. 6A, and $f(x) = a_1 \times x_1 + a_2 \times x_2 + a_3 \times x_3 + a_4$ may be expressed. Parameters $a_1$ to $a_4$ may be acquired from relationships between the feature vector acquired from existing data and calories.

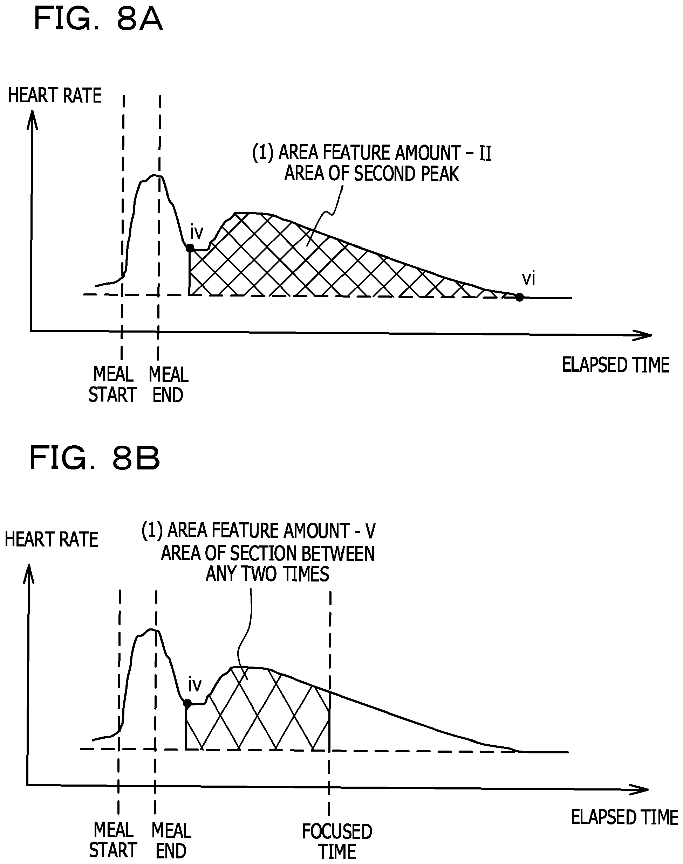

Estimated remaining calories may be calculated as the ingestion index. The estimated remaining calories are intake calories having not absorbed yet at a focused time of a total intake calories. For example, $(x_1, x_2)$ = (the area II of the second peak, the area from the start time of the second peak to the focused time) may be used as the feature vector x. FIG. 8A is a graph illustrating the area II of the second peak. FIG. 8B is a graph illustrating the area V from the start time of the second peak to the focused time. In this case, $f(x) = 1 - x_2/x_1$ may be expressed. The use of the function f enables estimated remaining calories at the focused time to be calculated.

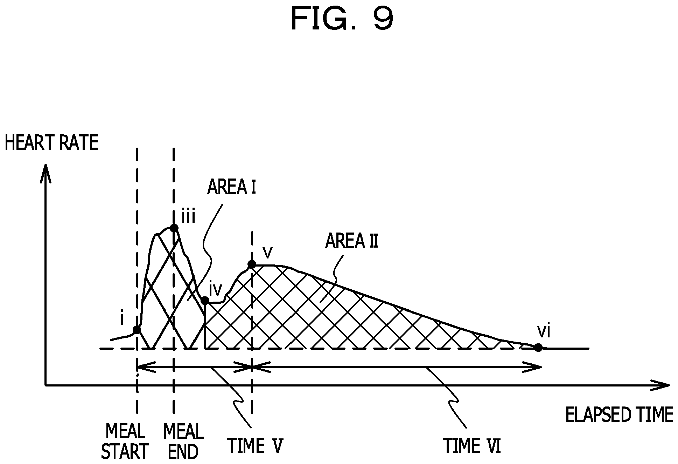

Estimated digestive power may be calculated as the ingestion index. Note that the digestive power represents an ability to digest. For example, if time taken to digest and absorb food is short despite a high index related to the total amount of eaten food, it can be said that the digestive power is high. Hence, for example, $(x_1, x_2, x_3, x_4)$ = (the area I, the area II, the time period V, the time period VI) may be used as the feature vector x. FIG. 9 is a graph illustrating these feature amounts. In a case where this feature vector x is used, $(x_1+x_2)/(x_3+x_4)$ may be used as the function f(x).

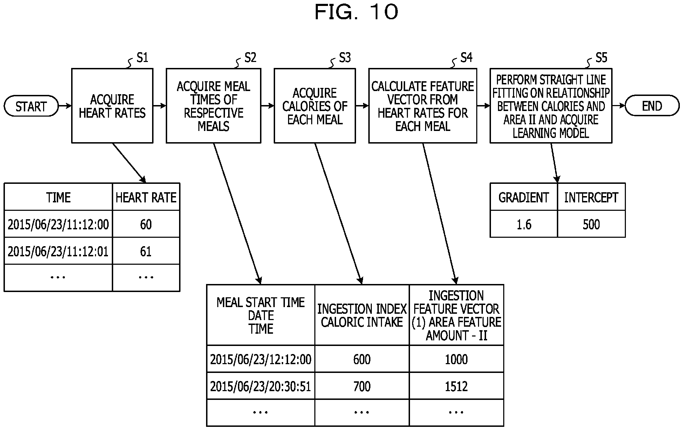

Subsequently, a specific example of calculating an estimated caloric intake will be described with reference to a flowchart. FIG. 10 is an example of a flowchart representing a process for calculating the function f. As illustrated in FIG. 10, the heart-rate acquisition unit 10 acquires heart rates from the biological-signal measurement apparatus 105 (step S1). For example, the heart-rate acquisition unit 10 acquires a heart rate every minute.

Subsequently, the feature-point extraction unit 20 acquires meal times of respective meals (step S2). Each meal time includes at least one of a meal start time and a meal end time. Subsequently, the ingestion-index estimation unit 40 acquires the calories of each meal (step S3). For example, the ingestion-index estimation unit 40 acquires calories input by the user. Subsequently, the feature-vector calculation unit 30 calculates a feature vector from the heart rates for each meal (step S4). The area II is herein calculated. Subsequently, the ingestion-index estimation unit 40 performs straight line fitting on a relationship between the calories and the area II by using the least squares method for each meal and acquires the gradient and intercept of the fitted line (step S5). Performing the steps in the flowchart enables a learning model of the relationship between the caloric intake and the area II to be acquired in advance.

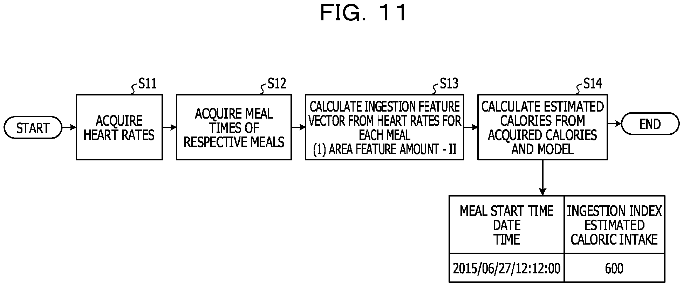

Subsequently, a specific example in which a caloric intake is estimated by using the acquired learning model will be described with reference to a flowchart. FIG. 11 is an example of a flowchart representing a process for estimating a caloric intake. As illustrated in FIG. 11, the heart-rate acquisition unit 10 acquires heart rates from the biological-signal measurement apparatus 105 (step S11). Subsequently, the feature-point extraction unit 20 acquires meal times of the respective meals (step S12). For example, the feature-point extraction unit 20 acquires each meal time of the corresponding meal after extracting the feature points i to vi. Subsequently, the feature-vector calculation unit 30 calculates a feature vector for each meal from the heart rates (step S13). The area II is herein calculated. Subsequently, the ingestion-index estimation unit 40 calculates estimated calories by applying the area II to the learning model acquired in advance (step S14).

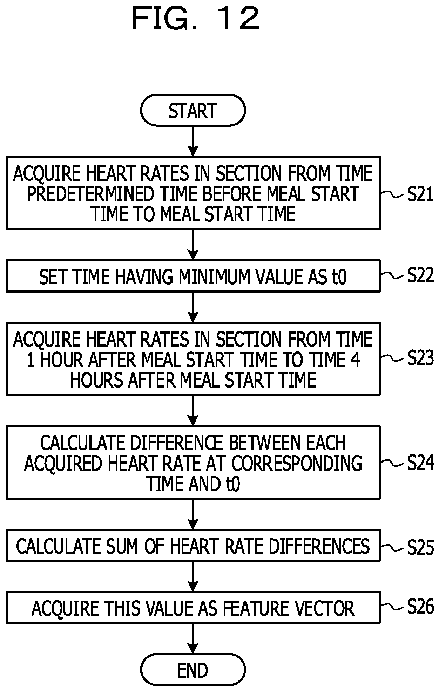

FIG. 12 is an example of a flowchart representing a process for calculating the area II. As illustrated in FIG. 12, the feature-vector calculation unit 30 acquires, from the heart-rate acquisition unit 10, heart rates in a section from time a predetermined time before (for example, 15 minutes before) the meal start time to the meal start time (step S21). The feature-vector calculation unit 30 then sets time having the minimum value of the heart rates acquired in step S21 as t0 (step S22). Step S22 is processing for extracting the feature point i.

The feature-vector calculation unit 30 then acquires, from the heart-rate acquisition unit 10, heart rates in the section from the feature point iv after the meal start time (for example, one hour after the meal start time) to the feature point vi (for example, four hours after the meal start time) (step S23). The feature-vector calculation unit 30 may thereby acquire heart rate data regarding the section from the feature point iv to the feature point vi. The feature-vector calculation unit 30 then calculates a difference between each acquired heart rate at the corresponding time and the heart rate at the time t0 (step S24). The feature-vector calculation unit 30 then calculates the sum of the calculated heart rate differences (step S25). The feature-vector calculation unit 30 acquires the value calculated in step S25 as the area II (step S26).

According to this embodiment, an index related to ingestion is estimated in meal-induced time variation of a biological signal value by using the feature amounts of the time variation of the biological signal value after the end of a meal. In this case, meal-induced variation of the biological signal value over a long span of time is used. That is, biological signal value variation induced by a digestive event, absorption, or the like is used. The accuracy of the ingestion index estimation may thereby be enhanced.

Calculating an integration value (the area) in the time variation of the biological signal value after the meal end enables the calculated value to be used as a feature amount. Calculating at least one of a rising speed and a lowering speed in a section between a point in time variation of the biological signal value and a point after the meal end in the time variation of the biological signal value enables the calculated value to be used as a feature amount. Calculating at least one of a rising range and a lowering range in a section between a point in time variation of the biological signal value and a point after the meal end in the time variation of the biological signal value enables the calculated value to be used as a feature amount. Calculating a time length of a section between a point in time variation of the biological signal value and a point after the meal end in the time variation of the biological signal value end enables the calculated value to be used as a feature amount. Calculating a plurality of function values based on a case where at least one of the above-described feature amounts is the sum of the plurality of function values enables the calculated value to be used as a new feature amount.

(Caloric Intake Evaluation Example)

For a comparison purpose, a caloric intake is estimated by using a peak area from the meal start to the meal end. Although a correlation between a peak area and a caloric intake is obtained to a certain extent, the correlation coefficient has a small value. That is, only a low correlation is obtained. In contrast, in a case where a caloric intake is estimated by using the area II after the meal end, the correlation coefficient of the correlation between the caloric intake and the area II has a value 1.5 to 2 times larger than the value in the comparative case. That is, a higher correlation is obtained. It is conceivable that the use of meal-induced variation of the biological signal value over a long span of time leads to enhancement of the accuracy of the ingestion index.

(Different Example of Meal-induced Time Variation of Heart-rate)

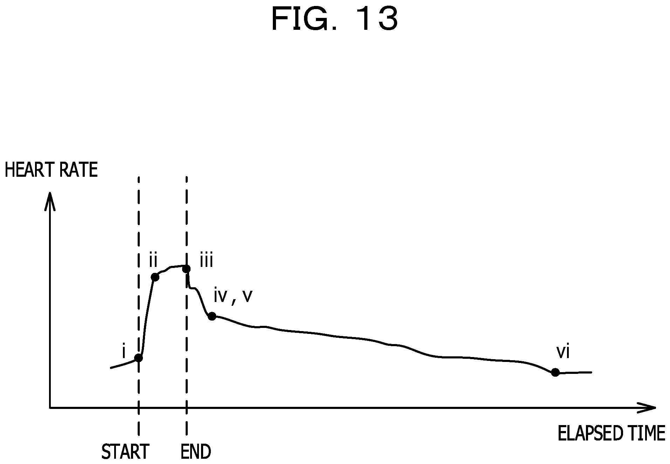

FIG. 13 is a graph illustrating a different example of meal-induced time variation of a heart rate. As illustrated in FIG. 13, there is a case where the local maximum point of the second peak does not appear in the heart-rate-rising section (heartbeat peak). In this case, the feature point iv at which the decrease of the heart rate is settled in the first peak and the feature point v at which the gentle decrease of the high level heart rate after the meal end is started are approximately identical. Hence, in the case where the second peak does not appear as in FIG. 13, the feature point iv and the feature point v may be used on the assumption that the feature point iv and the feature point v are the same time.

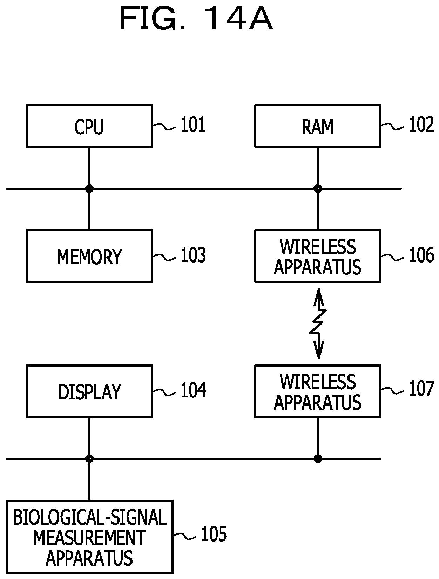

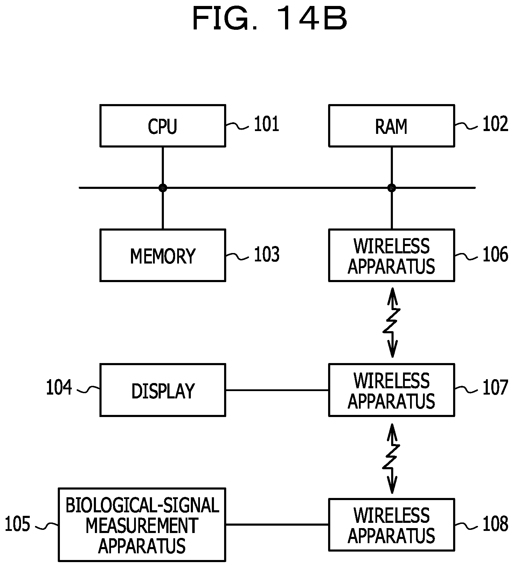

FIGs. 14A and 14B are each a diagram illustrating a different apparatus configuration of a corresponding one of the ingestion-index estimation apparatus 100 and an ingestion-index estimation apparatus 100a. As illustrated in FIG. 14A, an ingestion-index estimation apparatus may be configured such that a server and a wearable device wirelessly exchange data, the server including the CPU 101, the RAM 102, the memory 103, and a wireless apparatus 106, the wearable device including the display 104, the biological-signal measurement apparatus 105, and a wireless apparatus 107. In addition, as illustrated in FIG. 14B, the ingestion-index estimation apparatus may be configured such that a server, a terminal, and a wearable device wirelessly exchange data, the server including the CPU 101, the RAM 102, the memory 103, and the wireless apparatus 106, the terminal including the display 104 and the wireless apparatus 107, the wearable device including the biological-signal measurement apparatus 105 and a wireless apparatus 108.

Note that in the embodiment described above, the feature-point extraction unit 20 and the feature-vector calculation unit 30 each function as an example of a feature-amount extraction unit that extracts a feature amount of time variation of a biological signal value after the end of a meal in meal-induced time variation of the biological signal value. The ingestion-index estimation unit 40 functions as an example of an index estimation unit that estimates an index related to ingestion by using the feature amount extracted by the feature-amount extraction unit.

DESCRIPTION OF REFERENCE SIGNS

| 10 | heart-rate acquisition unit |
| 20 | feature-point extraction unit |
| 30 | feature-vector calculation unit |
| 31 | area-feature-amount calculation unit |
| 32 | speed-feature-amount calculation unit |
| 33 | amplitude-feature-amount calculation unit |
| 34 | time-feature-amount calculation unit |
| 35 | function-feature-amount calculation unit |
| 40 | ingestion-index estimation unit |
| 100 | ingestion-index estimation apparatus |

**Claims**

1. An information processing apparatus comprising:

   obtaining portion that obtains a feature amount of time series data indicating a time-dependent change of a biological signal value after a meal regarding the time series data of the biological signal value based on the meal, and
   determining portion that determins, based on the feature amount obtained by the obtaining portion, an index value related to the meal.

2. The information processing apparatus according to claim 1, wherein
   the obtaining portion obtains an integration value of the time series data of the biological signal value after the meal as the feature amount.

3. The information processing apparatus according to claim 1, wherein
   the obtaining portion obtains at least one of an increase speed and a decrease speed of the time-dependent change of the biological signal value between a first point of the time-dependent change of the biological signal value and a second point of the time-dependent change of the biological signal value after the meal as the feature amount.

4. The information processing apparatus according to claim 1, wherein
   the obtaining portion obtains at least one of an increase range and a decrease range of the time-dependent change of the biological signal value between a first point of the time-dependent change of the biological signal value and a second point of the time-dependent change of the biological signal value after the meal as the feature amount.

5. The information processing apparatus according to claim 1, wherein
   the obtaining portion obtains time range of the time-dependent change of the biological signal value between a first point of the time-dependent change of the biological signal value and a second point of the time-dependent change of the biological signal value after the meal as the feature amount.

6. The information processing apparatus according to claim 1, wherein
   the obtaining portion obtains values, the time-dependent change of the biological signal value being indicated as a sum of the plurality of function values.

7. The information processing apparatus according to claim 1, wherein
   the biological signal value indicates a heart rate.

8. The information processing apparatus according to claim 1, wherein the index value indicates at least one of a calorie included in the meal and a calorie related to metabolism.

9. A method executed by a computer, the method comprising:

   obtaining portion that obtains a feature amount of time series data indicating a time-dependent change of a biological signal value after a meal regarding the time series data of the biological signal value based on the meal, and
   determining portion that determines, based on the feature amount obtained by the obtaining portion, an index value related to the meal.

10. A program that causes a processor to execute operations, the operations comprising:obtaining portion that obtains a feature amount of time series data indicating a time-dependent change of a biological signal value after a meal regarding the time series data of the biological signal value based on the meal, and

determining portion that determines, based on the feature amount obtained by the obtaining portion, an index value related to the meal.

# FIG. 1

# FIG. 2A

```
┌─────────────┐    ┌─────────────┐    ┌─────────────┐    ┌─────────────┐
│   HEART-RATE │    │FEATURE-POINT │    │FEATURE-VECTOR│    │INGESTION-INDEX│
│  ACQUISITION │ →  │ EXTRACTION   │ →  │CALCULATION UNIT│ → │ESTIMATION UNIT│
│     UNIT     │    │    UNIT      │    │              │    │              │
└─────────────┘    └─────────────┘    └─────────────┘    └─────────────┘
      10                 20                 30                 40
```

# FIG. 2B

```
                    ┌──────────────────────────────┐ 30
                    │  ┌────────────────────────┐   │
                    │  │  AREA-FEATURE-AMOUNT    │ 31│
                    │  │   CALCULATION UNIT      │   │
                    │  └────────────────────────┘   │
                    │  ┌────────────────────────┐   │
                    │  │  SPEED-FEATURE-AMOUNT   │ 32│
                    │  │   CALCULATION UNIT      │   │
                    │  └────────────────────────┘   │
                    │  ┌────────────────────────┐   │
                    │  │  AMPLITUDE-FEATURE-     │ 33│
                    │  │ AMOUNT CALCULATION UNIT │   │
                    │  └────────────────────────┘   │
                    │  ┌────────────────────────┐   │
                    │  │  TIME-FEATURE-AMOUNT    │ 34│
                    │  │   CALCULATION UNIT      │   │
                    │  └────────────────────────┘   │
                    │  ┌────────────────────────┐   │
                    │  │  FUNCTION-FEATURE-      │ 35│
                    │  │ AMOUNT CALCULATION UNIT │   │
                    │  └────────────────────────┘   │
                    └──────────────────────────────┘
```

# FIG. 3A

HEART RATE

ELAPSED TIME

START  END

# FIG. 3B

HEART RATE

ii  iii

v

i  iv

vi

START  END

ELAPSED TIME

# FIG. 4A

HEART RATE

ELAPSED TIME

START END

# FIG. 4B

HEART RATE

ELAPSED TIME

START END

# FIG. 5A

# FIG. 5B

# FIG. 5C

FIG. 6A

FIG. 6B

# FIG. 7A

HEART RATE

II

iv

vi

ELAPSED TIME

MEAL   MEAL
START  END

# FIG. 7B

HIGH

CALORIE [kcal]

ESTIMATED CALORIES

LOW

SMALL

AREA II

LARGE

# FIG. 8A

HEART RATE

(1) AREA FEATURE AMOUNT – II
AREA OF SECOND PEAK

iv

vi

MEAL
START

MEAL
END

ELAPSED TIME

# FIG. 8B

HEART RATE

(1) AREA FEATURE AMOUNT - V
AREA OF SECTION BETWEEN
ANY TWO TIMES

iv

MEAL
START

MEAL
END

FOCUSED
TIME

ELAPSED TIME

# FIG. 9

## FIG. 10

| START | → | S1 ACQUIRE HEART RATES | → | S2 ACQUIRE MEAL TIMES OF RESPECTIVE MEALS | → | S3 ACQUIRE CALORIES OF EACH MEAL | → | S4 CALCULATE FEATURE VECTOR FROM HEART RATES FOR EACH MEAL | → | S5 PERFORM STRAIGHT LINE FITTING ON RELATIONSHIP BETWEEN CALORIES AND AREA II AND ACQUIRE LEARNING MODEL | → | END |

| TIME | HEART RATE |
|---|---|
| 2015/06/23/11:12:00 | 60 |
| 2015/06/23/11:12:01 | 61 |
| ... | ... |

| GRADIENT | INTERCEPT |
|---|---|
| 1.6 | 500 |

| MEAL START TIME DATE TIME | INGESTION INDEX CALORIC INTAKE | INGESTION FEATURE VECTOR (1) AREA FEATURE AMOUNT - II |
|---|---|---|
| 2015/06/23/12:12:00 | 600 | 1000 |
| 2015/06/23/20:30:51 | 700 | 1512 |
| ... | ... | ... |

# FIG. 11

```
           S11              S12                S13                  S14
┌─────────┐ ┌──────────┐ ┌──────────┐ ┌──────────────────────┐ ┌──────────────────┐ ┌─────┐
│  START  │→│ ACQUIRE  │→│ ACQUIRE MEAL │→│ CALCULATE INGESTION  │→│ CALCULATE ESTIMATED │→│ END │
│         │ │  HEART   │ │  TIMES OF    │ │ FEATURE VECTOR FROM  │ │   CALORIES FROM     │ │     │
│         │ │  RATES   │ │ RESPECTIVE   │ │ HEART RATES FOR      │ │ ACQUIRED CALORIES   │ │     │
│         │ │          │ │   MEALS      │ │   EACH MEAL          │ │    AND MODEL        │ │     │
│         │ │          │ │              │ │ (1) AREA FEATURE     │ │                     │ │     │
│         │ │          │ │              │ │     AMOUNT - II      │ │                     │ │     │
└─────────┘ └──────────┘ └──────────┘ └──────────────────────┘ └──────────────────┘ └─────┘
```

| MEAL START TIME DATE TIME | INGESTION INDEX ESTIMATED CALORIC INTAKE |
|---|---|
| 2015/06/27/12:12:00 | 600 |

# FIG. 12

```
        ( START )
            │
            ▼
┌─────────────────────────────────────┐
│ ACQUIRE HEART RATES IN SECTION FROM  │
│ TIME PREDETERMINED TIME BEFORE MEAL  │──── S21
│ START TIME TO MEAL START TIME        │
└─────────────────────────────────────┘
            │
            ▼
┌─────────────────────────────────────┐
│ SET TIME HAVING MINIMUM VALUE AS t0  │──── S22
└─────────────────────────────────────┘
            │
            ▼
┌─────────────────────────────────────┐
│ ACQUIRE HEART RATES IN SECTION FROM  │
│ TIME 1 HOUR AFTER MEAL START TIME TO │──── S23
│ TIME 4 HOURS AFTER MEAL START TIME   │
└─────────────────────────────────────┘
            │
            ▼
┌─────────────────────────────────────┐
│ CALCULATE DIFFERENCE BETWEEN EACH    │
│ ACQUIRED HEART RATE AT CORRESPONDING │──── S24
│ TIME AND t0                          │
└─────────────────────────────────────┘
            │
            ▼
┌─────────────────────────────────────┐
│ CALCULATE SUM OF HEART RATE          │──── S25
│ DIFFERENCES                          │
└─────────────────────────────────────┘
            │
            ▼
┌─────────────────────────────────────┐
│ ACQUIRE THIS VALUE AS FEATURE VECTOR │──── S26
└─────────────────────────────────────┘
            │
            ▼
        ( END )
```

# FIG. 13

# FIG. 14A

CPU ～101       RAM ～102

MEMORY ～103       WIRELESS APPARATUS ～106

DISPLAY ～104       WIRELESS APPARATUS ～107

BIOLOGICAL-SIGNAL MEASUREMENT APPARATUS ～105

# FIG. 14B

## INTERNATIONAL SEARCH REPORT

| International application No. |
| --- |
| PCT/JP2015/081829 |

**A. CLASSIFICATION OF SUBJECT MATTER**
*G06Q50/22*(2012.01)i

According to International Patent Classification (IPC) or to both national classification and IPC

**B. FIELDS SEARCHED**

Minimum documentation searched (classification system followed by classification symbols)
G06Q50/22

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | JP 10-504739 A  (Laniado, Eran Meia),<br>12 May 1998 (12.05.1998),<br>page 7, lines 2 to 25; page 9, lines 17 to 20<br>& US 5398688 A<br>1st row, line 45 to 2nd row, line 12; 3rd row,<br>lines 42 to 47<br>& WO 1996/003076 A1      & EP 0771173 A1<br>& AU 1918795 A            & CA 2195547 A1 | 1,3-5,7-10<br>2,6 |
| A | JP 2015-176213 A  (National University<br>Corporation Shizuoka University),<br>05 October 2015 (05.10.2015),<br>paragraph [0005]<br>(Family: none) | 1-10 |

☒  Further documents are listed in the continuation of Box C.          ☐  See patent family annex.

| | |
| --- | --- |
| * Special categories of cited documents:<br>"A" document defining the general state of the art which is not considered    to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    09 December 2015 (09.12.15) | Date of mailing of the international search report<br>    22 December 2015 (22.12.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

| International application No. |
| --- |
| PCT/JP2015/081829 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2010-229648 A (TOTO Ltd.), 14 October 2010 (14.10.2010), paragraphs [0001] to [0007] (Family: none) | 1-10 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10504739 A